# EUROPEAN PATENT SPECIFICATION

(11) **EP 0 575 800 B1**
(45) Date of publication and mention of the grant of the patent: **24.10.2001**
(21) Application number: 93109075.7
(22) Date of filing: 05.06.1993
(51) Int. Cl.: G01T 1/161, G01T 1/24

(54) **Flexible radiation probe**
Flexible Strahlungssonde
Sonde de rayonnement flexibles

(30) Priority: 12.06.1992 US 898074
(43) Date of publication of application: 29.12.1993
(73) Proprietor: THOMSON AND NIELSEN ELECTRONICS LIMITED, Ottawa, Ontario K2C 3P1 (CA)
(72) Inventor: Thomson, Ian, Nepean, Ontario K2H 8C4 (CA)
(74) Representative: Caron, Gérard

(56) References cited:
- EP-A- 0 471 957
- CA-A- 1 204 885
- US-A- 4 976 266
- IEEE TRANS ON NUCLEAR SCIENCE, vol. ns32, no. 6, December 1985, NY USA, pages 4446-4452, XP002010452 POSEY,LD ET AL.: "MOS-TRANSISTOR RADIATION DETECTORS AND X-RAY DOSE-ENHANCEMENT EFFECTS"

## Description

### Field of the Invention

This invention relates to flexible packaging for miniature metal oxide semiconductor field effect transistor (MOSFET) and more particularly to packaging for a MOSFET sensor probe used as a radiation dosimeter.

### Background of the Invention

The use of MOSFETs for detecting radiation is well known. It is understood that the threshold voltage of a MOSFET, or as it is sometimes called an insulated gate field effect transistor (IGFET) varies with exposure to radiation and therefore provides a useful building block in the design of dosimeters. The theory behind the use of these MOSFET dosimeters has been described in a number of papers some of which have been authored by the inventor. A paper entitled "Radiation Dosimetry with MOS Sensors" by Ian Thomson, R.E. Thomas and L Berndt, published in "Radiation Protection Dosimetry", VoL 6, No. 1, pp. 121-124, December, 1983, and a paper entitled "Semiconductor MOSFET Dosimetry", published in the proceedings of the Health Physics Society 1988 Annual Meeting presents the theory behind MOSFET dosimeters, as well as experimental results with response to radiation of different kinds. These papers provide useful information on the background of this application.

Various configurations of MOSFETs have been implemented in the prior art in order to measure the amount of radiation dose received, while at the same time overcoming the numerous problems which limit the accuracy and stability of these devices. A recent implementation of a direct reading dosimeter using IGFETs is described in the inventor's United States Patent No. 5,117,113 dated May 26, 1992. This patent discloses a radiation dosimeter having a pair of IGFETs integrated into the same silicon substrate, in which each of the transistors are operable in a bias mode and a test mode. A circuit element is provided for determining, during the test mode, the difference in the threshold voltages of the transistors, whereby the difference voltage is indicative of the radiation dose, and a circuit element is provided for continuously switching the transistors between the bias mode and the test mode, whereby the period of operation of the transistors in the test mode time period is small in comparison to the period of operation of the transistors in the bias mode.

There are a number of applications that require the use of a dosimeter to measure radiation. The sensitivity of a MOSFET to radiation is dependent upon the thickness of the gate oxide and the gate bias. By placing a small voltage, example 3 to 10 volts, on the gate of a MOSFET the sensitivity of the sensor can be enhanced.

MOSFETs of the type disclosed in the US. patent 5,117,113 would have to be mounted on a suitable package or substrate. Generally this takes the form of a standard IC package such as an 8 pin dual in line package (DIP). This type of packaging is adequate for the applications such as portable dosimeters or even badge type dosimeters.

A standard 8 pin DIP packaging is too bulky in uses such as in vivo radiation measurement. It is generally desirable for the dosimeter to be attached to a patient or inserted into a patient. In the latter case, this may require the use of a catheter where sterile conditions apply. United States Patent No. 4,976,266 to Huffman et al. discloses a method and apparatus for in vivo radiation measurements which uses a MOSFET dosimeter. A disadvantage of the Huffman device is that it requires the separation of the MOSFETs by having a single MOSFET in a probe and another matched MOSFET outside the probe in order to provide temperature compensation. A compensation circuit is connected with this matched unirradiated MOSFET to operate at a current designed to eliminate temperature dependence of the device. However the human body is a much higher temperature than ambient temperature and it is likely that Huffman does not achieve the temperature compensation which is required of this type of application since any flexing of the catheter, particularly near the MOSFET, is likely to break the lead wire to the MOSFET. A further disadvantage with the Huffman device is the mounting of the MOSFET within the catheter with an epoxy which itself is limited in application. Further, the MOSFET is rigidly mounted which further limits the possible uses of the dosimeter. Since the threshold voltage of the MOSFET increases with cumulative dose and cannot be re-set to its original value, the dosimeter is generally discarded after a number of exposures. In the case of current MOSFET dosimetry systems when a saturation level of 20,000 cGy is reached the dosimeter is discarded. This means that for radiotherapy exposures of 200 cGy, the dosimeter may be used up to 100 times before it is discarded. In the case of higher dose exposures, such as in bracytherapy, the dosimeter may be used only 20 times or less.

A major disadvantage of Huffman and current devices is that they are not intended for large scale manufacture and thus the cost of these dosimeters does not justify use on a routine basis.

Furthermore there is a requirement for radiation workers to wear dosimeters at their extremities where these extremities are likely to receive higher doses than their whole body badges. Examples include technicians who work in the manufacture of radioisotopes, and technicians in nuclear medicine departments of hospitals who administer radioisotopes, physicians and nurses who work in the X-ray beam with fluoroscopy procedures and nuclear plant workers.

The annual dose allowed to extremities is 50 rems, as opposed to 2 reins for whole body since the extremities are less susceptible to negative effects of radiation than the organs in the body. The current requirements for lowest detectable levels for extremity dosimeters is 250 mrem (0.25 rem,) and the maximum is 10 rem as determined by the "US Department of Energy-Standard for the Performance Testing of Extremity Dosimetry" draft May 4, 1991 (Rev 4). (Radiotherapy dose units used earlier were in cGy and we assume for simplicity that 1 rem = 1 cGy.)

The most commonly used extremity dosimeter is a TLD, which is fixed to the wrist or fingers of the worker with adhesive tape or special finger ring type holders. Some disadvantages of this approach are:
(i) TLD extremity dosimeters suffer from the same drawbacks as other TLD dosimeters in that they can only be read with a special laboratory. instrument. The crystals must be removed from their holders and manually handled. This is a relatively expensive slow process and, once read, the dose information is erased. In addition, identification can be lost once the crystal is removed from its holder which may contain an identification tag. In some applications, daily dose readings are carried out, thus requiring the facility to have twice as many dosimeters as workers since reading is carried out in a laboratory.
(ii) Extremity TLD dosimeters are generally too large to wear at the fingertips, which is the area of highest dose in most handling operations. A finger ring or wrist type is most commonly used in this application.
(iii) One of the main types of radiation of interest for extremity dosimetry is beta particles. These particles travel a short distance in material. Placement on the wrist or on a finger ring will not give an accurate measure of the dose to finger tips or thumbs. The dose at the wrist, for example, may be orders of magnitude different from that at the thumb.
(iv) Normal TLD crystals are too thick to adequately measure all energies of beta particles. The performance of TLD dosimeters is, therefore, not uniform with different types of radiation such as beta particles and X-rays. There also exists the need for a dosimeter which can be attached to a person's extremities (e.g. fingers, hands, head,) to measure personal radiation dose.

There therefore still exists a need for a miniature dosimeter that is capable of being used, for example, in vivo radiation measurement or which can be bent into any configuration to conform the sensor to its measuring environment. The sensor must also be capable of compensating for a wide variation in temperature and must also be sufficiently cost effective to justify its large scale use.

There are also times when it may be inconvenient to have many wires leading from the flexible circuit to direct reading circuitry. For instance, in radiotherapy, it is desirable to keep material on the patient to a minimum. At present thermoluminescence TLDs are used to measure the dose received by a patient. However, they often take hours to read and consequently pose further discomfort to an already uncomfortable situation.

There also exists a need in, for example, radiosurgery for a dosimeter which has high spatial resolution.

### Summary of the Invention

This invention seeks to provide packaging for a MOSFET, which is relatively easy to manufacture and which also exhibits a high degree of spatial resolution, while at the same time being extremely flexible.

In accordance with this invention there is provided a flexible radiation probe comprising:
a pair of insulated gate field effect transistors integrated into the same semiconductor substrate each having a gate, source and drain; and
an elongate flexible member for supporting the transistors at a first end thereof, and for electrically connecting the gate, source and drains of each of the transistors to a second end remote to the first end, the second end being adapted for connection to external circuitry.

A further embodiment provides for a radiation dosimeter comprising:
flexible member having a plurality of electrical connection tracks extending between first and second ends thereof;
a semiconductor radiation sensor supported on the first end of the flexible member, the sensor having a pair of insulated gate field effect transistors integrated into the same substrate each having a gate, source and drain, and the electrical connection tracks for connecting to a respective one of the source, drain and gate; and
means connectable to the second end of the flexible member for differentially biasing the transistors so that one of the transistors is more sensitive to ionising radiation than the other of the transistors during exposure of the transistors to radiation.

A further feature provides for a method for monitoring of radiation applied to a body comprising the steps of:
(i) inserting into the body a flexible radiation probe having a flexible member including a plurality of electrical connection tracks extending between flrst and second ends thereof and a radiation sensor supported at a first end of the flexible member, the sensor having a pair of insulated gate field effect transistors integrated into the same substrate each having a gate, source and drain, the electrical connection tracks connecting the source, drain and gate to the second end;
(ii) periodically biasing the transistors differentially and reading out the differential threshold voltage between the transistor through the flexible member.

### Brief Description or the Drawings

These and other features of the invention will become apparent, by way of example, from the following description in which reference is made to the appended drawings wherein:
FIGURES 1(a) and (b) show a top and side view, respectively, of a flexible sensor according to the present invention;
FIGURE 1(c) shows a magnified view of part of the flexible sensor as shown in Figures 1(a) and (b);
FIGURE 2 is an electrical schematic diagram showing electrical connections for a pair of IGFETs according to the present invention;
FIGURE 3(a) is a top view of a semiconductor die which is mounted according to the present invention;
FIGURE 3(b) is a schematic drawing showing bonding of leads to an IGFET, according to the present invention;
FIGURE 4(a) is a cross-sectional view of a packaging for a semiconductor;
FIGURE 4(b) is a further embodiment of a semiconductor packaging;
FIGURE 5(a) is a schematic drawing of a passive dosimeter using a plurality of flexible sensors according to the present invention;
FIGURE 5(b) is an electrical schematic drawing showing the connection of a pair of IGFETs for a passive dosimeter;
FIGURE 6(a) is a schematic top view of an extremity dosimeter;

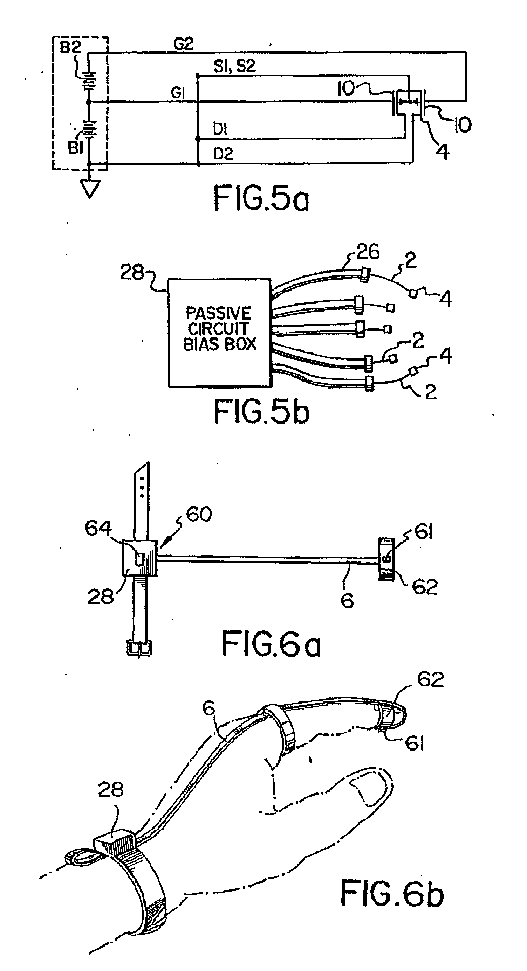

FIGURE 6(a) is a schematic view showing a use of the dosimeter of Figure 6(a);

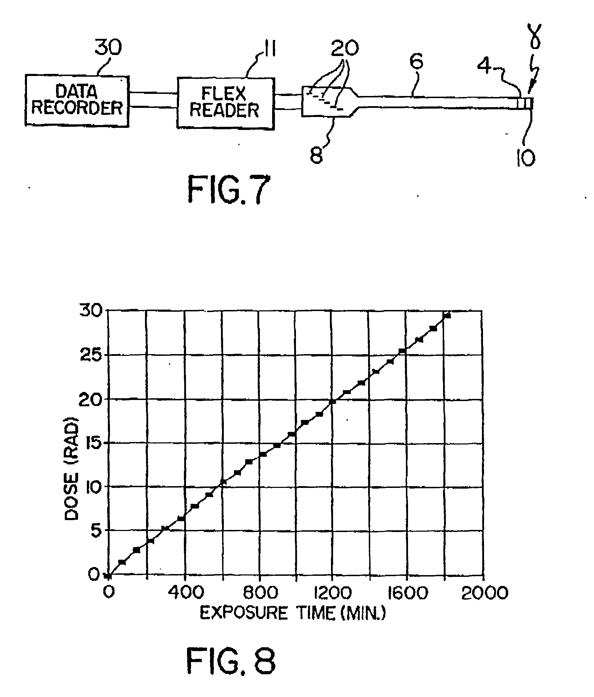

FIGURE 7 is a schematic drawing of a direct reading dosimeter according to an embodiment of the present invention; and
FIGURE 8 is a graph showing the correlation between a dose measured by a direct dosimeter as a function of exposure time for a dosimeter according to the present invention.

### Description of a Preferred Embodiment

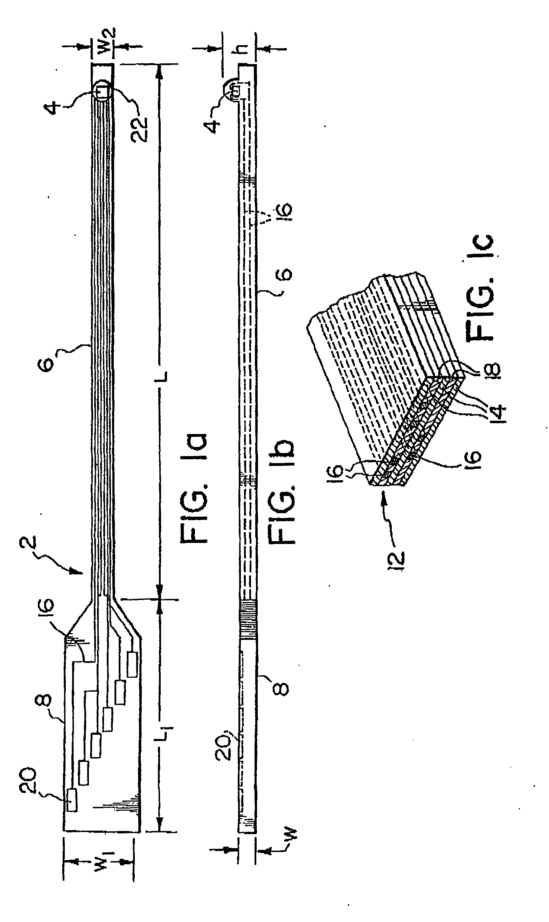

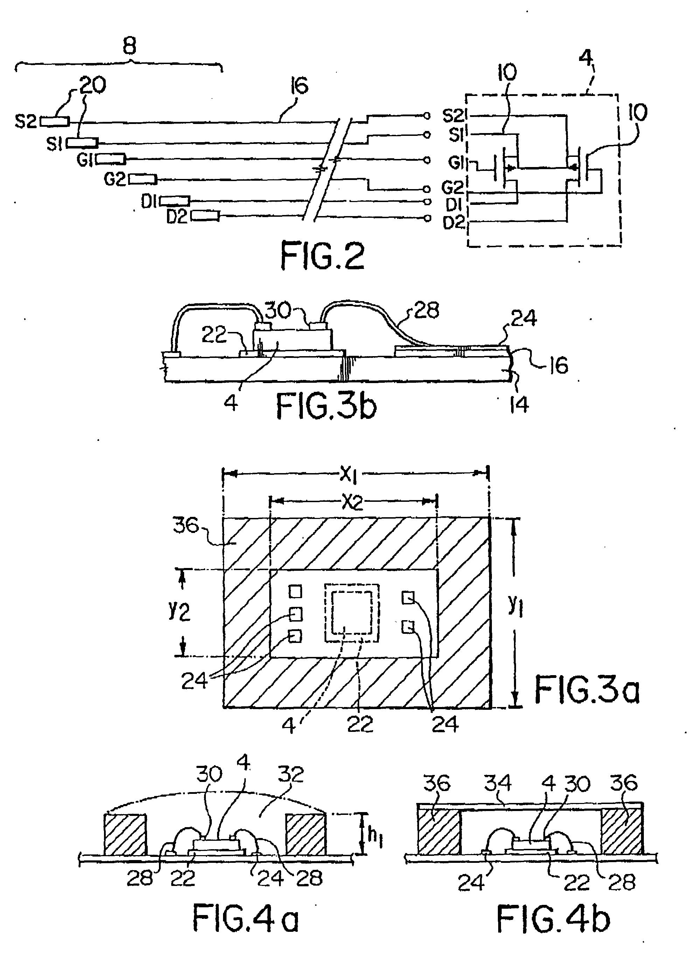

Referring to Figures 1(a) and (b) a flexible radiation probe is shown generally by numeral 2. The probe has a radiation sensor 4, which is supported on a first end of an elongated flexible member or circuit board 6. The radiation sensor 4 is comprised of a pair of IGFET 10 transistors shown schematically in Figure 2. These transistors are preferably of the same type and are fabricated in the same semiconductor die in order that they may have the same temperature variation characteristics, the same substrate resistivity and the same slow surface states prior to radiation. Each of the IGFETs 10 have a gate, source and drain terminals and are labelled G1, S1, D1 and G2, S2, and D2, respectively. The common substrate is connected internally in the IGFETs to their sources S1 and S2.

Referring back to Figures 1(a), (b) and more particularly Figure 1(c), the flexible circuit board 6 is composed of a multilayer sandwich structure of alternately superposed polyimide film 14 and layers of copper connection tracks 16. Each layer of the sandwich structure is held together by epoxy 18. Polyimide film is used in the preferred embodiment, however any other suitable material which may be bent a number of times without damage may be used. A suitable polyimide film is kapton™. Each of the conductive copper tracks 16 are each bonded to a respective one of the IGFET terminals as shown in Figure 3(b). The conductive copper tracks 16 are gold plated at least on an upper surface in the vicinity of the first end to form bonding pads 24 so that wires 28 may be bonded from these tracks 16 to aluminum metallization pads 30 on the top surface of the IGFET die. The bonding wire 28 is generally 25µm diameter, gold or aluminum. The flexible circuit 6 is enlarged at a second end 8 remote to the first end. In order that the probe may be connected to suitable external circuitry, a plurality of connector pads 20 are provided thereat Each pad is 5 mm long by 1 mm wide and connects to a respective one of the copper tracks. The above described arrangement is also shown schematically in Figure 2.

For the probe to be used in catheters or the like the flexible circuit must be made as small as possible. Thus the width w₂ of flexible circuit board 6 is preferably not much larger than the width of the radiation sensor 4 which itself is less than 2.2 mm on edge. A preferred length L of the flexible circuit 6 may be varied depending on where it is to be placed and how the radiation probe 2 is to be used. If, for example, the flexible circuit 6 is to be placed in the catheter (not shown) for use in brachytherapy, the flexible circuit may be 10 to 15 cm in length. It is preferred that the thickness w of flexible circuit 6 be very small, and at least less than .3 mm.

In the embodiment shown in Figures 1(a), (b) and (c) the dimensions for the flexible radiation probe 2 are:
L = 24.4 cm
w = 0.03 cm
h = 0.1 cm
L₁ = 4.4 cm
w₁ = 0.69 cm
w₂ = 0.22 cm

The sensor 4 is generally covered with epoxy, which increases the height h of the sensor 4 to approximately 0.1 cm. In the preferred embodiment the copper conducting tracks 16 are composed of 0.002 - 0.01 cm electrodeposited or rolled/annealed copper. The IGFET semiconductor chip die 4 is epoxied to a 0.025 cm² gold pad 22 which in turn is bonded to a rigid circuit board material. Five gold bonding pads 24, each 0.003 cm² in area, 1µm in thickness, surround the IGFET as shown in Figure 3(a). Once the semiconductor die 10 has been epoxied into place onto the gold pad 22, wire bond 28 connections are made from the semiconductor metallization pads 30 to the respective bonding pads 24 and the semiconductor is covered with an epoxy 32, such as hysol™ as shown cross-section in Figure 4(a).

The epoxy covered sensor of Figure 4(a) is adequate for receiving x-rays and gamma rays, however in some applications such as for example in measuring (beta particle) radiation to the skin of a human body this packaging is inadequate.

A suitable packaging for beta particle measurement is shown in Figure 4(b). One key requirement for beta particle measurement is that the sensor must measure the dose behind a shield which has the equivalent thickness of (70 µm of tissue) which is equivilant to 7 mg/cm2. The average thickness of the epidermis (outer layer of human skin) is 70 µm.

This is generally achieved by placing the IGFETs 10 in a package having a foil lid 34. As described earlier, the semiconductor die is bonded to the end of the flexible material and wire bond connections are made as before. In addition thereto an annular wall 36 is constructed around the semiconductor die to form a "well" by bonding insulating material such as printed circuit board to the flexible circuit as shown in Figure 4(b) and Figure 3(a). The foil lid 34 generally comprising aluminum or metallized Mylar™ of appropriate thickness is bonded to the top of the wall, in order to protect the chip and provide a means for beta particles to enter the package.

The IGFETs are known to respond to beta particles in the same way as other types of radiation and over a wide range of energies. This is because of the extremely thin active region of the device (the silicon dioxide is < 1µm thick) thus beta particles down to 20 keV can be detected with unshielded dosimeters. Thick dosimeters such as TLDs have poorer performance with low energy beta particles. The thinnest TLD crystals commercially available for extremity dosimetry are about 25*µ*m thick.

The extremity dosimeter previously described can be used to monitor skin dose from X-rays and gamma rays, but can also be simplified for these applications. The packaging as shown in Figure 4(a) is less expensive to make than using a 70µm window of Figure 4(b) and may be used in applications where it is known that only X-rays or gamma rays exist In nuclear medicine, for example, over 90% of the radiation exposure is due to the use of Tc99m, which emits X-rays at 140 keV. A simple epoxy covered sensor with or without a "well" is perfectly adequate for this single-use application.

The typical dimensions for a sensor as shown in Figure 3(a) are:
x₁ = 0.274"
x₂ = 0.174"
y₁ = 0.185"
y₂ = 0.085"
h₁ = 0.05"

In use, the IGFETs are exposed to radiation while one transistor is biased relative to the other transistor and it has been found that more charge will accumulate upon radiation under the gate of the biased transistor compared to that of the unbiased transistor, thereby shifting its threshold voltage by a greater amount. By measuring the difference in threshold voltages it is possible to determine the radiation received by the IGFETs 10. In practice, an external circuit 11, as shown in Figure 7, provides the bias for the IGFETs 10. This circuit 11 operates the IGFETs in a bias mode and a read mode. A circuit and method for performing the read and bias mode operations is described in the inventor's United States Patent No. 5,117,113.

The IGFETs 10 are in bias mode 97% of the time, making the sensor sensitive to radiation. The other 3% of the time the sensor is placed into a read mode. In this manner, a direct reading can be taken and the radiation dose can be monitored in real time. This would be of particular interest when a radiotherapy machine is to be checked for beam flatness. The medical physicist often has to shape the beam using collimators. This is often time consuming using ionisation chambers or thermoluminescence devices (TLDs). It is envisioned that the flexible dosimeter connected to direct reading circuitry will replace these TLDs and increase the efficiency of the beam shaping process. An array of flexible probes 2 may be used to monitor the beam in real time and irradiation iterations could be performed without interruptions.

There are several advantages to using a direct reading circuit. The most apparent is decreased time spent reading absorbed dose either to patients or phantoms. Traditionally TLDs are used however they take several hours to be read. With the flexible dosimeter connected to direct reading circuitry more patients can be treated thus increasing the efficiency of the radiotherapy clinic. Another advantage is that the direct reading circuit could also be interfaced with a data recorder or a computer and absorbed dose could be monitored and recorded. This would be useful when a patient has been implanted with either radioactive beads or wires. Often the implant may last for a number of days with doses of several thousand of rads. Currently the inventor is not aware of any direct-reading method to measure absorbed dose for these patients. Post radiation stability may also be enhanced by using a test/bias circuit.

The read/bias circuitry requires a large number of wires (six) leading from the flexible probe 2 to the read/bias circuitry. There are times however when it is more convenient to have few wires leading from the flexible radiation probe 2 to the read/bias circuitry. For instance, in radiotherapy, it is desirable to keep the material on the patient to a minimum. Figures 5(a) and (b) show a configuration for this application. The IGFETs 10 are placed in their most sensitive mode by placing a small bias voltage on the gate G1 and G2, respectively of each IGFET 10. A bias source such as a hearing aid battery B1 and B2 are connected in series with the positive terminal of a battery B2 connected to the gate G2 of a IGFET 10 and with the other terminal of the battery B1 connected to the gate G1 of the other IGFET 10. The sources S1 and S2 and drains D1 and D2 are connected together as shown schematically in Figure 5(b). With the IGFETs 10 in their bias mode there is no current to the gate oxide thus the lifetime of the batteries B1 and B2 is essentially their shelf life. After irradiation, the flexible radiation probe 2 is disconnected from the bias batteries B1 and B2 and is connected to a a readout circuit (not shown). The read out circuit may provide the user with the dose received by the IGFETs 10 and its past radiation history (example cumulative dose). In order to increase the sensitivity of the pair to measure lower doses, the differential bias is increased by means of more batteries from typically 6V/3V to 15V/3V. This increases the sensor differential sensitivity from 1 mV/rem to approximately 4 mV/rem thus increasing the signal to the reader.

There are applications which require multiple passive flexible radiation probes. In this instance as shown in Figure 5(a) a passive circuit bias box 24 is used to provide the similar bias levels of the batteries B1 and B2 of Figure 5(b). Each of the multiple flexible radiation probes 2 may be connected via a suitable cable 26 to the passive circuit bias box 24.

In the case of an extremity dosimeter for personal use, the bias box may also include a semiconductor memory device (not shown) such as an EEPROM. This device only requires power when it is being read from or written to. The memory may be used to permanently store data such as:
(i) Identification of the dosimeter and, if required, the user. Where dosimeters are not shared by different workers, the wearer's employee number or other unique identifier is written into this memory.
(ii) The dose history of the dosimeter, so that this information may be read by any other reader and is not reader specific.
(iii) Calibration information for the dosimeter, such as the exact calibration factor (mV/rem).
(iv) Other data which may be required to calculate dose or identify the wearer or location of the dosimeter.
This configuration of the flexible radiation probe 2 is known as a passive flexible radiation probe.

In the case of an extremity dosimeter as shown in Figures 6(a) and 6(b), a relatively small passive bias box 28 is attached to the second end 60 of the flexible member 6. The bias box is typically attached to the wrist for band extremities, but may also be attached to other parts of the body for other extremities (e.g. leg, head or feet). The sensor end 61 is positioned on the extremity using the tabs 62 to fasten around a finger as shown in Figure 6(b). The position of the sensor at the end of the flexible member can be determined by the user e.g. finger tip, knuckle joint etc. In many cases, a protective glove (not shown) will also be worn on top of the dosimeter and this helps to keep the dosimeter in place.

In this configuration the dosimeter is used as follows. The dosimeter and its bias box 28 are electrically connected by a connector 64 on the bias box to the reader (not shown) which measures the differential threshold voltage of the MOSFET pair as described previously. The dosimeter is then worn for a period of time, typically a work period of one day, and the device is read again. The difference in the differential threshold voltages is proportional to the radiation dose received by the dosimeter. A dosimeter user can carry out a reading in less than 1 minute in the field, thus requiring only one dosimeter per worker.

The reader for this application is different from that for radiotherapy in that it must be able to read smaller differential MOSFET voltages than is required for radiotherapy since the doses in extremity dosimetry are much smaller than in therapy. This means that, with 4 mV/rem, we require to measure 0.8 mV (i.e. 800 uV) to correspond to 200 mrems. In practice, an electronic reading instrument of the type described for the radiotherapy system with increased voltage amplification can read to 5 uV, which is more than adequate as this corresponds to approximately 1 mrem.

The reader must also be capable of measuring the bias on the MOSFETs and indicating if the batteries require changing. It must read and write the information described above in the EEPROM memory device and print this out to give a hardcopy and/or transfer it electronically to another device such as a computer.

A simplified version of the reading circuitry (not shown) may also be included in the bias box 28 so that the wearer may get a direct reading of the radiation dose. This may have advantages in potentially dangerous radiation fields where an alarm may be required. This would mean that the reading circuitry would require only a differential threshold measurement and alarm function. The main reader would still be used for all other functions. This approach would not be used in all applications as the normal mode is to leave the dosimeter and glove on until the end of a work period.

### Beam Quality Checks

Cobalt-60 and linear accelerators are often used in radiotherapy. Traditionally, ionization chambers, Thermoluminescence Dosimeters and/or films are most often used to measure the beam profile. Good spatial resolution of absorbed dose measurement is needed, and in the measurement of high absorbed dose gradients it is essential. It is in this area that the IGFET dosimeter has its greatest advantages over the above mentioned technologies. The IGFET sensor 4 area can be made less than 1mm by 1mm. It is envisioned that an array of flexible circuits can be positioned to measure the profile of a radiation beam in real time. This would greatly enhance the efficiency of the beam profiling process.

### Phantom Desimetry

In radiotherapy, the specification of the complete absorbed dose distribution within the radiation beam in a phantom is a prerequisite for calculating the prescribed absorbed dose to the target volume in the patient. It is envisioned that the direct reading flexible radiation probe 2 can be implanted at various locations in a phantom to measure absorbed dose. With this method the phantom does not have to be disassembled. The IGFETs can be read while still in the phantom and further beam modifications can take place.

### In Vivo

In vivo measurements can be divided into five classes.

### (i) Entrance absorbed dose.

These are mainly to check the machine output, the absorbed dose distribution across the patient and the positioning of shielding in relation to the position of the patient The excellent spatial resolution of the flexible IGFET sensor may be exploited in this instance and greatly reduce set up time and enhance beam time efficiency.

### (ii) Intra-cavity absorbed dose measurements

The absorbed dose within a body cavity (e.g. the mouth, nasopharynx, vagina, rectum, etc) can be measured using the flexible radiation probe 2. The flexible probe 2 may, if required for sterility reasons, first be placed within a catheter (not shown) and then placed within the body cavity (not shown). The flexible probes 2 are useful in that they are temperature independent, as described in U.S. Patent 5,117,113. In this manner the flexible dosimeter can be read in real time or placed as a passive dosimeter and read periodically.

### (iii) Individual spared organ absorbed dose measurements.

The absorbed dose to spared (shielded) organs can be measured using the flexible probe 2. The flexible probe 2 can be placed in and around the shield to measure the absorbed dose. Once again the sensor 4 can be read in real time or as a passive beam check.

### (iv) Radiosurgery.

Radiosurgery is a technique that employs accurate stereotactic localization of intracranial targets and allows accurate deposition of a single high dose of radiation to the target volume, while minimizing dose to the surrounding brain structure. Very small volumes are treated with radiosurgery ranging from less than 1 cc to a few cc. This ability of precise tumour and treatment localization and the use of small beams creates steep dose gradients. It is possible to measure absorbed dose using a flexible probe 2 and circuit. It is very important that tissue just a few millimetres from the targeted volume receives insignificant dose. The inventor is not aware of other direct-reading dosimeters on the market at this time that are capable of the spatial resolution that is needed for radiosurgery. To date patient's planned treatments are calculated through computer simulations.

### (v) Conductive interstitial hyperthermia.

The goal for hyperthermia is to raise tumour temperature above a cytotoxic threshold for an extended period of time without harmful elevation of surrounding normal tissue temperatures. The flexible probe 2 can be used to measure absorbed dose when the patient is undergoing hyperthermia treatments. The response of the flexible probe 2 is independent of temperature, and is therefore ideal for this procedure.

The flexible probe 2 may be used in the same manner as standard packaged MOSFET dosimeters with the following perceived advantages:
(i) The spatial resolution of the flexible probe dosimeter has been substantially improved. The sensor 2 area (MOS dosimeter plus surrounding package) is 2.2mm x 2mm. The flexible circuit is 1mm thick while its present length is 24.4 cm. The size of the flexible dosimeter makes dosimetry for radiosurgery possible.
(ii) The flexible probe dosimeter is capable of being inserted into a catheter. The very nature of the flexible circuit allows it to be bent into many configurations. It is therefore possible to place the sensitive area of the flexible dosimeter within areas of the body that were heretofore difficult to reach.
(iii) The sensitive area of the flexible dosimeter can also be placed within a water tank without fear of destroying the MOS dosimeter. Since the flexible dosimeter has been designed to be placed within the body for in vivo measurements its advantage over other types of dosimetry is its humidity independence.
(iv) The flexible probe can be configured as a passive dosimeter or as a direct reading dosimeter. The advantage of the passive dosimeter is that there are a reduced number of cables connecting it to a readout circuit, thus minimizing the material that is placed within or near the radiation beam. In direct reading mode the radiation beam can be monitored in real time. This has the advantage of decreasing the set up time for patient planning. Absorbed dose can be monitored at a specific point within a phantom, and the beam can be modified and subsequent measurements can be made without disassembling the phantom.
(v) Beam quality checks can be made with the flexible circuit placed in an array. The configuration of the array is left to the user.

It has been found that the radiation performance of the flexible probes 2 with respect to sensitivity, linearity, temperature dependence, and dose rate are similar to that of other packaged MOS devices and more particularly as described in U.S. Patent No. 5,117,113. Referring to Figure 8, results of a direct reading flexible probe are shown where the radiation data was recorded in a data recorder 30 as shown in Figure 7.

While the invention has been described in connection with a specific embodiment thereof and in a specific use, various modifications thereof will occur to those skilled in the art without departing from the scope of the invention as set forth in the appended claims.

The terms and expressions which have been employed in the specification are used as terms of description and not of limitations, and there is no intention in the use of such terms and expressions to exclude any equivalents of the features shown and described or portions thereof, but it is recognized that various modifications are possible within the scope of the claims to the invention.

## Claims

1. Radiation probe (2) comprising :
a pair of insulate gate field effect transistors (10) integrated into the same semiconductor substrate each having a gate (G1, G2), source (S1, S2) and drain (D1, D2),
said radiation probe (2) being **characterised by** :
an elongate circuit board (6) capable of being bent into many configurations, said elongate circuit board (6) having :
- a first end for supporting said transistors (10);
- a second end remote to said first end, said second end being adapted for connection to external circuitry (11); and
- a plurality of electrically conductive tracks (16) extending between said first and second ends for electrically connecting said gate (G1, G2), source (S1, S2) and drain (D1, D2) of each of said transistors (10) to said second end.

2. Probe as defined in claim 1, said first end including a rigid member (14) for mounting said semiconductor thereon.

3. Probe as defined in claim 2, said first end including a plurality of bonding pads (24) being arranged around said semiconductor and being attached to said rigid member (14), said source (S1, S2), drain (D1, D2) and gate (G1, G2) of each of said transistors (10) being connected to a respective one of said bonding pads (24).

4. Probe as defined in claim 3, said semiconductor and said bonding pads (24) being covered with a suitable epoxy (32).

5. Probe as defined in claim 1, said semiconductor being surrounded along its edges by a wall (36) of insulation material.

6. Probe as defined in claim 5, including a metal cover (34) arranged over the top surface of said semiconductor and being supported by said wall (36).

7. Probe as defined in claim 6, said metal cover (34) being metallised mylar.

8. Probe as defined in claim 6, said metal cover (34) being a foil of aluminium.

9. Probe as defined in claim 6, said metal cover (34) having a thickness of 7 mg/cm².

10. Probe as defined in claim 1, said external circuitry (11) including means for differentially biasing said transistors (10) and means for reading a differential threshold voltage between said transistors (10).

11. Probe as defined in claim 1, said tracks (16) being copper.

12. Probe as defined in claim 1, said tracks (16) each being 0.01 inches wide and having a thickness in the range of 0.002 to 0.01 cm.

13. Probe as defined in claim 1, said second end being enlarged and including a plurality of connection pads (20), each electrically connected to a respective on of said tracks (16).

14. Probe as defined in claim 1, said elongate circuit board (6) being a multilayer circuit board.

15. Probe as defined in claim 14, in which said multilayer circuit board is composed of alternately superposed layers of polyimide film (14) and layers of said connection tracks (16).

16. Probe as defined in claim 15, said polyimide being Kapton.

17. Probe as defined in claim 1, in which the width of said elongate circuit board (6) is smaller than a diameter of a catheter so that said radiation probe (2) may be inserted into said catheter.

18. Radiation dosimeter comprising :
a radiation probe (2) according to claim 1; and
means (28) differentially biasing said transistors (10) connectable to said second end of said elongate circuit board (6) for so that one of said transistors (10) is more sensitive to ionising radiation that the other of said transistors (10) during exposure of said transistors (10) to radiation.

19. Dosimeter as defined in claim 18, including reader means connectable to said second end of said elongate circuit board (6) for reading out the differential threshold voltage between said transistors (10) after irradiation, said differential threshold voltage being indicative of the radiation received by said transistors (10).

20. Dosimeter as defined in claim 18, said biasing means being a battery.

21. Dosimeter as defined in claim 18, said biasing means including a memory means for storing user information and dosimeter information.

22. Dosimeter as defined in claim 21, said memory means being an EEPROM.

23. Dosimeter as defined in claim 21, said reader including a visual display for displaying said radiation dose.

24. Dosimeter as defined in claim 18, said elongate circuit board (6) including a fastening tab (62) at said first end for attaching said first end to a predetermined position on body.

25. Radiation dosimeter comprising :
a radiation probe (2) according to claim 1; and
means connectable to said second end for periodically biasing said transistors (10) differentially and reading out the differential threshold voltage between said transistors (10) through said elongate circuit board (6).

26. Method for monitoring of radiation comprising the steps of :
a) inserting into the body a radiation probe (2) according to claim 1;
b) differentially biasing said transistors (10) by a bias source connected to said second end of said elongate circuit board (6) through said elongate circuit board (6) during irradiation;
c) disconnecting said bias source from said second end of said elongate circuit board (6) after predetermined time period; and
d) reading out the differential threshold voltage between said transistors (10) by a read out means connected to said second end of said elongate circuit board (6).

27. Method as defined in claim 26 wherein said probe is inserted into a catheter.

28. Method for monitoring of radiation applied to a body comprising the steps of :
a) inserting into the body a radiation probe (2) according to claim 1; and
b) periodically biasing said transistors (10) differentially and reading out the differential threshold voltage between said transistor through said elongate circuit board (6).

29. Method of claim 28 wherein said probe is inserted into a catheter.

30. Method of claim 29 wherein a plurality of radiation sensors each mounted on elongate circuit board (6) are spaced a predetermined distance apart so as to measure the profile of a radiation beam.

31. Method for monitoring of radiation applied to an extremity of a body comprising the steps of :
a) attaching to said body a radiation probe (2) according to claim 1;
b) attaching said first end with said sensor, to a said extremity by way of a fixing tab (62);
c) connecting said second end to a means for differentially biasing said transistors (10); and
d) reading out the differential threshold voltage between said transistors (10) by a reader means, said differential threshold voltage being indicative of a radiation dose received at said extremity.

32. Method as defined in claim 31, including a metal cover (34) arranged over the top surface of said semiconductor and being supported by a wall (36).

33. Method as defined in claim 32, said metal cover (34) being metallised mylar.

34. Method as defined in claim 32, said metal cover (34) being a foil of aluminium.

35. Method as defined in claim 32, said metal cover (34) having a thickness of 7 mg/cm².

36. Method as defined in claim 31, said semiconductor being covered with a suitable epoxy (32).

## Patentansprüche

1. Strahlungssonde (2), umfassend:
ein Paar Isolierschicht-Feldeffekttransistoren (10), die in dasselbe Halbleitersubstrat integriert sind und jeweils ein Gate (G1, G2), eine Source (S1, S2) und einen Drain (D1, D2) besitzen,
wobei die Strahlungssonde (2) **gekennzeichnet ist durch**:
eine langgestreckte Leiterplatte (6), die in viele Konfigurationen gebogen werden kann und umfaßt:
- ein erstes Ende zum Tragen der Transistoren (10);
- ein zweites Ende, das vom ersten Ende entfernt und so beschaffen ist, daß es an eine externe Schaltungsanordnung (11) angeschlossen werden kann; und
- mehrere elektrisch leitende Bahnen (16), die sich zwischen dem ersten Ende und dem zweiten Ende erstrecken, um das Gate (G1, G2), die Source (S1, S2) und den Drain (D1, D2) jedes der Transistoren (10) mit dem zweiten Ende elektrisch zu verbinden.

2. Sonde nach Anspruch 1, wobei das erste Ende ein starres Element (14) enthält, um darauf den Halbleiter anzubringen.

3. Sonde nach Anspruch 2, wobei das erste Ende mehrere Kontaktierungsflächen (24) enthält, die um den Halbleiter angeordnet und an dem starren Element (14) befestigt sind, wobei die Source (S1, S2), der Drain (D1, D2) und das Gate (G1, G2) jedes der Transistoren (10) mit einer entsprechenden der Kontaktierungsflächen (24) verbunden sind.

4. Sonde nach Anspruch 3, wobei der Halbleiter und die Kontaktierungsflächen (24) mit einem geeigneten Epoxid (32) bedeckt sind.

5. Sonde nach Anspruch 1, wobei der Halbleiter längs seiner Kanten von einer Wand (36) aus Isoliermaterial umgeben ist.

6. Sonde nach Anspruch 5, die eine Metallabdeckung (34) enthält, die über der oberen Fläche des Halbleiters angeordnet und durch die Wand (36) getragen ist.

7. Sonde nach Anspruch 6, wobei die Metallabdeckung (34) mit Metall beschichtetes Mylar ist.

8. Sonde nach Anspruch 6, wobei die Metallabdeckung (34) eine Aluminiumfolie ist.

9. Sonde nach Anspruch 6, wobei die Metallabdeckung (34) eine Dicke von 7 mg/cm² hat.

10. Sonde nach Anspruch 1, wobei die externe Schaltungsanordnung (11) eine Einrichtung zum differentiellen Vorspannen der Transistoren (10) und eine Einrichtung zum Lesen einer differentiellen Schwellenspannung zwischen den Transistoren (10) enthält.

11. Sonde nach Anspruch 1, wobei die Bahnen (16) Kupfer sind.

12. Sonde nach Anspruch 1, wobei die Bahnen (16) jeweils eine Breite von 0,01 Zoll und eine Dicke im Bereich von 0,002 bis 0,01 cm besitzen.

13. Sonde nach Anspruch 1, wobei das zweite Ende verbreitert ist und mehrere Anschlußflächen (20) enthält, wovon jede mit einer entsprechenden der Bahnen (16) elektrisch verbunden ist.

14. Sonde nach Anspruch 1, wobei die langgestreckte Leiterplatte (6) eine Mehrschicht-Leiterplatte ist.

15. Sonde nach Anspruch 14, in der die Mehrschicht-Leiterplatte aus abwechselnd übereinanderliegenden Schichten aus einem Polyimid-Film (14) und Schichten der Anschlußbahnen (16) gebildet ist.

16. Sonde nach Anspruch 15, wobei das Polyimid Kapton ist.

17. Sonde nach Anspruch 1, in der die Breite der langgestreckten Leiterplatte (6) kleiner als ein Durchmesser eines Katheters ist, so daß die Strahlungssonde (2) in den Katheter eingeschoben werden kann.

18. Strahlungsdosimeter, umfassend:
eine Strahlungssonde (2) nach Anspruch 1; und
eine Einrichtung (28), die die Transistoren (10) differentiell vorspannt und an das zweite Ende der langgestreckten Leiterplatte (6) angeschlossen werden kann, so daß einer der Transistoren (10) für lonisierungsstrahlung empfindlicher als der andere der Transistoren (10) ist, während die Transistoren (10) Strahlung ausgesetzt sind.

19. Dosimeter nach Anspruch 18, das eine Leseeinrichtung enthält, die an das zweite Ende der langgestreckten Leiterplatte (6) angeschlossen werden kann, um die differentielle Schwellenspannung zwischen den Transistoren (10) nach der Bestrahlung auszulesen, wobei die differentielle Schwellenspannung die von den Transistoren (10) empfangene Strahlung angibt.

20. Dosimeter nach Anspruch 18, wobei die Vorspannungseinrichtung eine Batterie ist.

21. Dosimeter nach Anspruch 18, wobei die Vorspannungseinrichtung eine Speichereinrichtung zum Speichern von Benutzerinformationen und Dosimeterinformationen umfaßt.

22. Dosimeter nach Anspruch 21, wobei die Speichereinrichtung ein EEPROM ist.

23. Dosimeter nach Anspruch 21, wobei die Leseeinrichtung eine optische Anzeige zum Anzeigen der Strahlungsdosis umfaßt.

24. Dosimeter nach Anspruch 18, wobei die langgestreckte Leiterplatte (6) am ersten Ende einen Befestigungsstreifen (62) aufweist, um das erste Ende an einer vorgegebenen Position am Körper zu befestigen.

25. Strahlungsdosimeter, das umfaßt:
eine Strahlungssonde (2) nach Anspruch 1; und
eine Einrichtung, die mit dem zweiten Ende verbunden werden kann, um die Transistoren (10) periodisch differentiell vorzuspannen und um die differentielle Schwellenspannung zwischen den Transistoren (10) durch die langgestreckte Leiterplatte (6) auszulesen.

26. Verfahren zum Überwachen von Strahlung, das die folgenden Schritte umfaßt:
a) Einführen einer Strahlungssonde (2) nach Anspruch 1 in den Körper;
b) differentielles Vorspannen der Transistoren (10) mittels einer mit dem zweiten Ende der langgestreckten Leiterplatte (6) verbundenen Vorspannungsquelle über die langgestreckte Leiterplatte (6) während der Bestrahlung;
c) Trennen der Vorspannungsquelle vom zweiten Ende der langgestreckten Leiterplatte (6) nach einer vorgegebenen Zeitperiode; und
d) Auslesen der differentiellen Schwellenspannung zwischen den Transistoren (10) durch eine Ausleseeinrichtung, die mit dem zweiten Ende der langgestreckten Leiterplatte (6) verbunden ist.

27. Verfahren nach Anspruch 26, bei dem die Sonde in einen Katheter eingeschoben wird.

28. Verfahren zum Überwachen von Strahlung, mit der ein Körper beaufschlagt wird, das die folgenden Schritte umfaßt:
a) Einführen einer Strahlungssonde (2) nach Anspruch 1 in den Körper; und
b) periodisches differentielles Vorspannen der Transistoren (10) und Auslesen der differentiellen Schwellenspannung zwischen den Transistoren über die langgestreckte Leiterplatte (6).

29. Verfahren nach Anspruch 28, bei dem die Sonde in einen Katheter eingeschoben wird.

30. Verfahren nach Anspruch 29, bei dem mehrere Strahlungssensoren, wovon jeder auf einer langgestreckten Leiterplatte (6) angebracht ist, um eine vorgegebene Strecke voneinander beabstandet sind, um das Profil eines Bestrahlungsstrahls zu messen.

31. Verfahren zum Überwachen der Strahlung, mit der eine Extremität eines Körpers beaufschlagt wird, das die folgenden Schritte umfaßt:
a) Befestigen einer Strahlungssonde (2) nach Anspruch 1 am Körper;
b) Befestigen des ersten Endes mit dem Sensor an der Extremität mittels eines Befestigungsstreifens (62);
c) Verbinden des zweiten Endes mit einer Einrichtung zum differentiellen Vorspannen der Transistoren (10); und
d) Auslesen der differentiellen Schwellenspannung zwischen den Transistoren (10) durch eine Leseeinrichtung, wobei die differentielle Schwellenspannung eine bei der Extremität empfangene Strahlungsdosis angibt.

32. Verfahren nach Anspruch 31, mit einer Metallabdeckung (34), die über der oberen Fläche des Halbleiters angeordnet ist und durch eine Wand (36) getragen wird.

33. Verfahren nach Anspruch 32, wobei die Metallabdeckung (34) mit Metall beschichtetes Mylar ist.

34. Verfahren nach Anspruch 32, wobei die Metallabdeckung (34) eine Aluminiumfolie ist.

35. Verfahren nach Anspruch 32, wobei die Metallabdeckung (34) eine Dicke von 7 mg/cm² hat.

36. Verfahren nach Anspruch 31, wobei der Halbleiter durch ein geeignetes Epoxid (32) bedeckt ist.

## Revendications

1. Sonde de radiation (2) comprenant :
une paire de transistors à effet de champ à grille isolée (10) intégrés dans le même substrat semi-conducteur et comprenant chacun une grille (G1, G2), une source (S1, S2) et un drain (D1, D2),
ladite sonde de radiation (2) étant **caractérisée par** :
une plaquette de circuit allongée (6) susceptible d'être courbée dans plusieurs configurations, ladite plaquette de circuit allongée (6) ayant :
- une première extrémité pour supporter lesdits transistors (10) ;
- une seconde extrémité, éloignée de ladite première extrémité, adaptée pour être connectée à un circuit externe (11) ; et
- une pluralité de pistes électriquement conductrices (16) s'étendant entre lesdites première et seconde extrémités pour connecter électriquement ladite grille (G1, G2), ladite source (S1, S2) et ledit drain (D1, D2) de chacun desdits transistors (10) à ladite seconde extrémité.

2. Sonde selon la revendication 1, ladite première extrémité comprenant un élément rigide (14) sur lequel est monté ledit semi-conducteur.

3. Sonde selon la revendication 2, ladite première extrémité comprenant une pluralité de plages de connexion (24) arrangées autour dudit semi-conducteur et attachés au dit élément rigide (14), ladite source (S1, S2), ledit drain (D1, D2) et ladite grille (G1, G2) de chacun desdits transistors (10) étant connectés chacun à l'une correspondante desdites plages de connexion (24).

4. Sonde selon la revendication 3, ledit semi-conducteur et lesdites plages de connexion (24) étant couverts d'une résine époxy adéquate (32).

5. Sonde selon la revendication 1, ledit semi-conducteur étant entouré le long de ses bords par une paroi (36) de matériau isolant.

6. Sonde selon la revendication 5, comprenant un couvercle métallique (34) disposé au dessus de la surface supérieure dudit semi-conducteur et supporté par ladite paroi (36).

7. Sonde selon la revendication 6, ledit couvercle métallique (34) étant un mylar métallique.

8. Sonde selon la revendication 6, ledit couvercle métallique (34) étant une feuille d'aluminium.

9. Sonde selon la revendication 6, ledit couvercle métallique (34) ayant une épaisseur de 7 mg/cm².

10. Sonde selon la revendication 1, ledit circuit externe (11) comprenant des moyens pour polariser de manière différentielle lesdits transistors (10) et des moyens pour lire une tension de seuil différentielle entre lesdits transistors (10).

11. Sonde selon la revendication 1, lesdites pistes (16) étant en cuivre.

12. Sonde selon la revendication 1, lesdites pistes (16) ayant une largeur de 0.01 pouces et une épaisseur comprise dans une gamme de 0.002 à 0.01 cm.

13. Sonde selon la revendication 1, ladite seconde extrémité étant élargie et comprenant une pluralité de plages de contact (20) connectées électriquement chacune à l'une correspondante desdites pistes (16).

14. Sonde selon la revendication 1, ladite plaquette de circuit allongée (6) étant une plaquette de circuit multicouche.

15. Sonde selon la revendication 14, dans laquelle ladite plaquette de circuit multicouche est composée d'une superposition alternée de couches de film polyimide (14) et de couches comprenant lesdites pistes de connexion (16).

16. Sonde selon la revendication 15, ledit polyimide étant du Kapton.

17. Sonde selon la revendication 1, dans laquelle la largeur de ladite plaquette de circuit allongée (6) est inférieure au diamètre d'un cathéter de sorte que ladite sonde de radiation (2) peut être insérée dans ledit cathéter.

18. Instrument de mesure de radiation comprenant :
une sonde de radiation (2) selon la revendication 1 ; et
des moyens (28) pour polariser de manière différentielle lesdits transistors (10) susceptibles d'être connectés à ladite seconde extrémité de ladite plaquette de circuit allongée (6) de sorte que l'un desdits transistors (10) est plus sensible au rayonnement ionisant que l'autre desdits transistors (10) durant une exposition desdits transistors (10) à des radiations.

19. Instrument de mesure selon la revendication 18, comprenant des moyens de lecture susceptibles d'être connectés à ladite seconde extrémité de ladite plaquette de circuit allongée (6) pour lire la tension de seuil différentielle entre lesdits transistors (10) après irradiation, ladite tension de seuil différentielle étant représentative des radiations reçues par lesdits transistors (10).

20. Instrument de mesure selon la revendication 18, lesdits moyens de polarisation étant une pile.

21. Instrument de mesure selon la revendication 18, lesdits moyens de polarisation comprenant des moyens de mémorisation pour stocker des informations relatives à l'utilisateur et à l'instrument de mesure.

22. Instrument de mesure selon la revendication 21, lesdits moyens de mémorisation étant une EEPROM.

23. Instrument de mesure selon la revendication 21, ledit lecteur comprenant un affichage visuel pour afficher la dose de radiations.

24. Instrument de mesure selon la revendication 18, ladite plaquette de circuit allongée (6) comprenant une languette de fixation (62) à ladite première extrémité pour attacher cette première extrémité à une position prédéterminée du corps.

25. Instrument de mesure de radiations comprenant :
une sonde de radiation (2) selon la revendication 1 ; et
des moyens susceptibles d'être connectés à ladite seconde extrémité pour polariser périodiquement et de manière différentielle lesdits transistors (10) et pour lire la tension de seuil différentielle entre lesdites transistors (10) via ladite plaquette de circuit allongée (6).

26. Procédé de surveillance de radiations comprenant les étapes suivantes :
a) insérer dans le corps une sonde de radiation (2) selon la revendication 1 ;
b) polariser de manière différentielle lesdits transistors (10) au moyen d'une source de polarisation connectée à ladite seconde extrémité de ladite plaquette de circuit allongée (6) via ladite plaquette de circuit allongée (6) durant l'irradiation ;
c) déconnecter ladite source de polarisation de ladite seconde extrémité de ladite plaquette de circuit allongée (6) après une période de temps prédéterminée ; et
d) lire la tension de seuil différentielle entre lesdits transistors (10) à l'aide d'un moyen de lecture connecté à ladite seconde extrémité de ladite plaquette de circuit allongée (6).

27. Procédé selon la revendication 6, dans lequel la sonde est insérée dans un cathéter.

28. Procédé de surveillance de radiations appliquées à un corps comprenant les étapes suivantes :
a) insérer dans le corps une sonde de radiation (2) selon la revendication 1; et
b) polariser périodiquement et de manière différentielle lesdits transistors (10) et lire la tension de seuil différentielle entre lesdits transistors (10) via ladite plaquette de circuit allongée (6).

29. Procédé selon la revendication 28, dans lequel la sonde est insérée dans un cathéter.

30. Procédé selon la revendication 29, dans lequel une pluralité de sonde de radiation montée chacune sur une plaquette de circuit allongée (6) sont espacées l'une de l'autre d'une distance prédéterminée afin de mesurer un profile d'un faisceau de radiation.

31. Procédé de surveillance de radiations appliquées à une extrémité d'un corps comprenant les étapes suivantes :
a) attacher au corps la sonde de radiation (2) selon la revendication 1 au corps ;
b) attacher ladite première extrémité comprenant le détecteur à ladite extrémité du corps au moyen d'une languette de fixation (62) ;
c) connecter ladite seconde extrémité à un moyen pour polariser de manière différentielle lesdits transistors (10) ; et
d) lire la tension de seuil différentielle entre lesdits transistors (10) à l'aide d'un moyen de lecture, ladite tension de seuil différentielle étant représentative de la dose de radiations reçues à ladite extrémité du corps.

32. Procédé selon la revendication 31, dans lequel un couvercle métallique (34) est disposé sur la surface supérieure dudit semi-conducteur et supporté par une paroi (36).

33. Procédé selon la revendication 32, ledit couvercle métallique (34) étant un mylar métallique.

34. Procédé selon la revendication 32, ledit couvercle métallique (34) étant une feuille d'aluminium.

35. Procédé selon la revendication 32, ledit couvercle métallique (34) ayant une épaisseur de 7 mg/cm².

36. Procédé selon la revendication 31, ledit semi-conducteur étant couvert d'une résine époxy adéquate (32).
